# FASCICULE DE BREVET EUROPEEN

(11) **EP 1 010 411 B1**
(45) Date de publication et mention de la délivrance du brevet: **25.02.2004**
(21) Numéro de dépôt: 99490044.7
(22) Date de dépôt: 14.12.1999
(51) Int. Cl.: A61F 13/15, B30B 9/22, G01N 33/36, D06F 47/06

(54) **Dispositif pour la réalisation de tests sur des substrats absorbants**
Vorrichtung zur Prüfung von absorbierenden Substraten
Device for testing absorbent substrates

(30) Priorité: 17.12.1998 FR 9816271
(43) Date de publication de la demande: 21.06.2000
(73) Titulaire: Courtray, Franck, 59500 Douai (FR)
(72) Inventeur: Courtray, Franck, 59500 Douai (FR)
(74) Mandataire: Ecrepont, Robert

(56) Documents cités:
- EP-A- 0 022 044
- EP-A- 0 359 501
- EP-A- 0 692 232
- EP-A- 0 797 967
- EP-A- 0 797 968
- EP-A- 0 813 849
- EP-A- 0 887 055
- US-A- 4 041 737

## Description

L'invention se rapporte à un dispositif pour la réalisation de tests sur des substrats absorbants.

L'invention concerne un dispositif pour la réalisation de tests sur des substrats absorbants, notamment par application d'au moins un élément, dit élément de test, contre une face dudit substrat.

L'invention s'applique avantageusement, mais non exclusivement à un dispositif pour la réalisation de tests dits de remouillage, sur des matelas absorbants entrant dans la confection d'articles d'hygiène de type absorbant, ou sur les articles absorbants eux mêmes.

Par articles d'hygiène de type absorbant, on désigne des articles qui comprennent au moins un matelas de matériau absorbant, par exemple, capillairement absorbant, dont une face est destinée à être, au moins indirectement appliquée contre le corps d'une personne en vue de recueillir un fluide.

En terme de performance, les produits absorbants tels que les changes pour bébés, les produits pour incontinents ou les produits d'hygiène féminine sont évalués sous deux critères :
- capacité à absorber et retenir les fluides émis pendant l'utilisation, ce que l'on peut considérer comme prévention des fuites,
- capacité à isoler l'utilisateur des fluides émis, ce qui peut se traduire par la prévention de l'humidité.

Le développement constant des marchés des produits absorbants, oblige les industriels à une mesure permanente des performances respectives de leurs produits selon ces deux critères.

Si les panels consommateurs peuvent procurer des informations relativement précises et fiables en ce qui concerne le taux de fuites, ces études pour être valables au sens statistique, doivent être larges et sont donc très coûteuses.

Mais c'est surtout à cause des délais de réponse qui sont toujours très longs qu'il a été nécessaire de mettre au point des méthodes de laboratoire beaucoup plus rapides et plus économiques.

L'accès à des systèmes de mannequins tels que le « labservice capability tester » et les études permanentes des corrélations obtenues avec ce système et les panels consommateurs ont permis à de nombreux industriels d'obtenir rapidement des informations précises et fiables concernant le taux de fuites de leurs produits.

A contrario, la mesure de la protection contre l'humidité par panels est nettement plus complexe et nettement moins fiable.

Ceci étant lié, par exemple, aux facteurs de variabilité émanant des individus et des caractéristiques intrinsèques de leur peau.

D'autre part, si le paramètre « fuites » est très pragmatique (il y a fuites ou pas), il n'en est pas de même pour ce qui est de l'appréciation de l'humidité, qui est un paramètre partiellement subjectif (légèrement humide, humide, très humide ... comment quantifier objectivement cette performance ?).

Depuis de nombreuses années, l'évaluation de la « prévention contre l'humidité » se fait de deux manières distinctes, c'est à dire, in-vivo et en laboratoire.

En laboratoire, on réalise les tests selon deux concepts de mesure:
- la mesure dite de remouillage ou « rewet »,
- la mesure de l'humidité en surface ou humidité en contact.

Les tests réalisés in-vivo exploitent quant à eux directement les propriétés de la peau de la personne qui porte l'article, mais aussi les conditions d'usage normal desdits articles.

Il s'avère que les résultats des tests réalisés in-vivo, qui devraient mieux refléter le comportement des articles absorbants que les tests pratiqués en laboratoire, soulèvent en fait des difficultés d'interprétation et impliquent une importante population d'individus testés.

Les chercheurs se sont attachés à trouver une solution qui permette de réaliser des tests en laboratoire avec une corrélation importante par rapport aux tests réalisés in-vivo.

Les chercheurs ont travaillé à la mise au point d'un élément qui, destiné à être appliqué contre le substrat absorbant à tester, présente des propriétés comparables à celles de la peau de personnes.

Des éléments de test très perfectionnés, faisant par exemple, appel au collagène, ont été mis au point et sont aujourd'hui utilisés, tant pour la mesure dite remouillage ou "rewet" que pour la mesure de l'humidité en surface ou humidité en contact.

Tel que cela a été annoncé, le dispositif de l'invention concerne avantageusement, c'est à dire non limitativement, la mesure du remouillage.

Par test de remouillage, on désigne un test selon lequel:
- un échantillon de matelas absorbant reçoit une certaine quantité de liquide,
- l'échantillon est, pendant un temps déterminé, soumis à une pression également déterminée, de manière à assurer la répartition et/ou la distribution du liquide,
- au moins un élément en matériau absorbant, de masse déterminée, est ensuite appliqué contre une surface de l'échantillon et maintenu contre cette surface pendant un temps et avec une pression déterminés,
- au terme de l'étape d'application, chaque élément en matériau absorbant est pesé en vue de déterminer la quantité totale de liquide recueillie.

De nombreuses variantes de ce test existent, du fait par exemple, de la nature des éléments absorbants utilisés, de leurs dimensions et formes...

L'inventeur a constaté, d'une part, que les éléments en matériau absorbants utilisés sont généralement de fines feuilles de matériaux, tels des éléments découpés dans des feuilles de papiers filtres, de collagène et, d'autre part, appliqués sur les échantillons à tester au moyen de corps massifs.

De nos jours, les articles d'hygiène de type absorbant ont couramment une structure qui n'est pas uniforme.

Par exemple, ils peuvent présenter l'un des critères que sont :
- une face réceptrice de fluide qui est anatomiquement conformée, et
- une épaisseur et/ou une densité qui varie en fonction du point de mesure sur la face réceptrice.

Sur des articles de ce type, les tests de remouillage conduits selon la technique ancienne ne sont pas fiables, c'est à dire que, non seulement ils donnent des résultats différents sur des articles identiques mais encore, ils ne révèlent pas les indices effectifs de remouillage des articles testés.

Un résultat que l'invention vise à obtenir est un dispositif qui, tout en étant économique à réaliser, permet l'application effective d'éléments absorbants de test contre des articles absorbants dont la structure n'est pas uniforme.

A cet effet, l'invention a pour objet un dispositif pour la réalisation de tests sur des substrats absorbants, notamment par application d'au moins l'une des faces opposées du dit substrat contre une surface réceptrice,
ce dispositif étant notament caractérisé en ce qu'il comprend un moyen dont la fonction est d'assurer l'application étroite du substrat contre cette surface réceptrice, c'est à dire un moyen à même de déformer élastiquement au moins ce substrat pour qu'il s'appuie étroitement contre ladite surface réceptrice, le moyen d'application étroite comprenant :
- une paroi mince en matériau élastiquement déformable qui, d'étendue sensiblement supérieure à l'étendue de l'une quelconque des faces opposées du substrat à tester, présente elle même deux faces opposées dont,
   . une face, dite frontale, destinée à coopérer au moins indirectement avec une face de substrat, et
   . une face opposée, dite dorsale, destinée à recevoir des actions d'application de la face frontale vers le substrat,
- un moyen de production d'actions d'application contre la face dorsale de la paroi mince,
- une surface réceptrice orientée pour recevoir au moins indirectement le substrat en appui sous l'effet de la paroi mince.

L'invention sera bien comprise à la lecture de la description ci-après faite en regard du dessin à titre d'exemple non limitatif, en regard du dessin ci-annexé qui représente schématiquement :

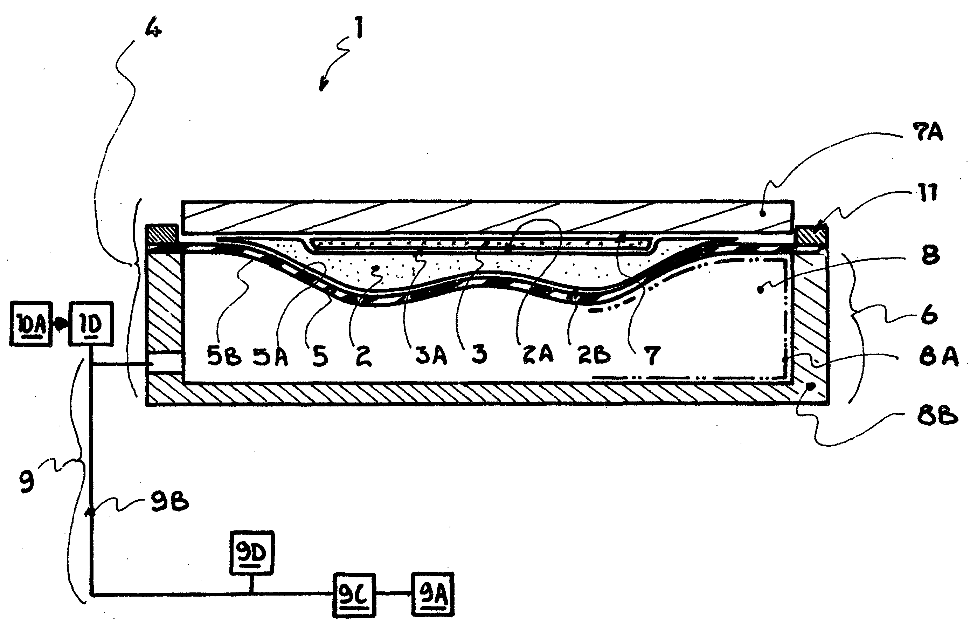

- figure 1 : un dispositif selon l'invention vu en coupe transversale,

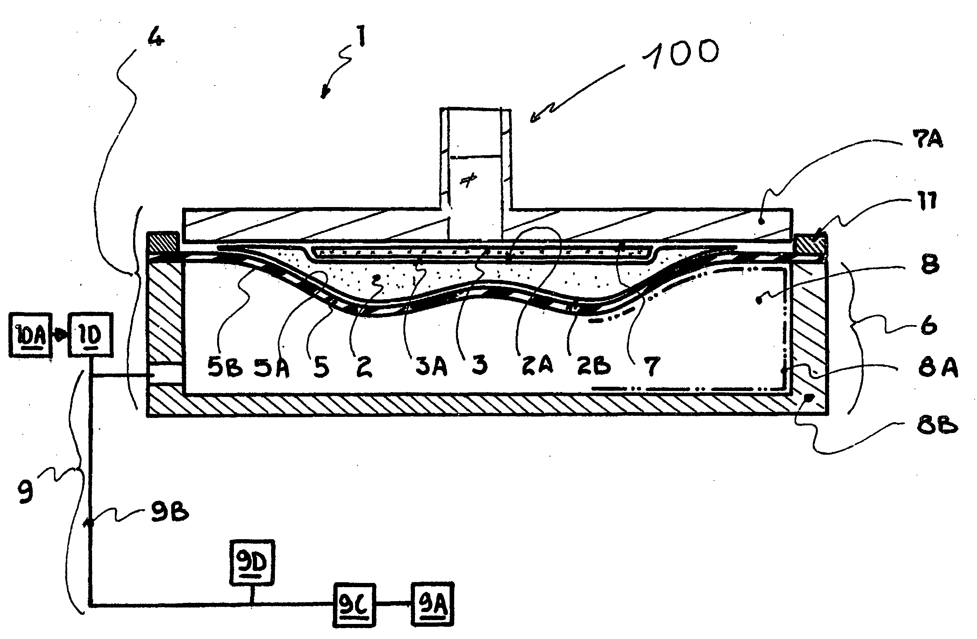

- figure 2 : une variante.

En se reportant au dessin, on voit un dispositif 1 pour la réalisation de tests sur des substrats absorbants 2, notamment par application d'au moins l'une des faces opposées 2A, 2B du dit substrat 2 contre une surface réceptrice 3A, 7, telle une face 3A d'au moins un élément 3, dit élément de test.

Avantageusement, mais non limitativement, !e dispositif permet la réalisation de tests dits de remouillage, sur de tels substrats.

Par substrats absorbants 2, on désigne plus particulièrement mais non exclusivement, les matelas absorbants qui entrent dans la confection d'articles d'hygiène de type absorbant, ou les articles absorbants eux mêmes.

De tels substrats 2 comprennent donc au moins un matelas de matériau absorbant, par exemple capillairement absorbant, dont une face 2A, dite réceptrice, est destinée à être, au moins indirectement appliquée contre le corps d'une personne (non représentée) en vue de recueillir un fluide.

Selon l'invention, le dispositif 1 est remarquable en ce qu'il comprend un moyen 4 dont la fonction est d'assurer l'application étroite du substrat 2 contre une surface réceptrice 3A, 7, c'est à dire un moyen 4 à même de déformer élastiquement au moins ce substrat 2 pour qu'il s'appuie étroitement contre ladite face réceptrice 3A, 7.

Lorsqu'une face 2A du substrat 2 doit être appliquée directement contre une surface réceptrice 7, le moyen 4 d'application étroite assure donc la déformation élastique dudit substrat 2 en vue de mettre effectivement la surface réceptrice 7 en contact avec la face 2A concernée de ce substrat 2.

Lorsqu'une face 2A du substrat 2 doit être appliquée contre une surface réceptrice 3A, telle une face 3A d'un élément 3 de test, le moyen 4 d'application étroite assure la déformation élastique dudit substrat et, le cas échéant de l'élément 3 de test, en vue de mettre effectivement la surface réceptrice 3A de cet élément 3 de test, en contact avec la face 2A concernée du substrat 2.

De manière remarquable, le moyen 4 d'application étroite est de type assurant une pression uniforme d'application du substrat absorbant 2 contre la surface réceptrice 3A, 7.

Notamment, le dispositif de l'invention permet donc que, lorsqu'un élément absorbant 3 est mis en oeuvre pour un test, il soit étroitement appliqué contre la face du substrat testé, même lorsque la zone d'application de l'élément 3 comprend des dépressions, c'est à dire sont en retrait du fait de la moindre épaisseur et/ou densité locale du substrat.

Avec les dispositifs de test de l'état de la technique qui mettaient en oeuvre au moins un corps massif et rigide pour l'application de chaque élément absorbant contre une face d'un substrat à tester, ce résultat ne pouvait être obtenu car ledit corps massif ne s'appuyait principalement que sur les parties saillantes de la face du substrat et accidentellement sur les zones en retrait de ces parties saillantes.

Le dispositif de l'invention comprenant un moyen 4 à même de déformer élastiquement au moins l'une des pièces 2, 3 que sont le substrat 2 et l'élément de test 3 pour qu'ils s'appuient étroitement par des faces 2A, 3A en contact, il permet donc de remédier aux inconvénients des dispositifs de test de l'état de la technique.

Dans une forme remarquable de réalisation, le moyen 4 d'application étroite comprend :
- une paroi mince 5 en matériau élastiquement déformable qui, d'étendue sensiblement supérieure à l'étendue de l'une quelconque des faces opposées 2A, 2B du substrat 2 à tester, présente elle même deux faces opposées 5A, 5B dont,
   . une face 5A, dite frontale, destinée à coopérer au moins indirectement avec une face de substrat 2, et
   . une face opposée 5B, dite dorsale, destinée à recevoir des actions d'application de la face frontale 5A vers le substrat 2,
- un moyen 6 de production d'actions d'application contre la face dorsale de la paroi mince 5,
- une surface réceptrice 7 orientée pour recevoir au moins indirectement le substrat 2 en appui sous l'effet de la paroi mince 5.

Dans une forme de réalisation, le dispositif comprend :
- une capacité 8 définie par une paroi 8A au moins localement constituée par la paroi mince 5,
- un moyen 9 commandé d'injection d'un fluide sous une pression déterminée dans cette capacité 8,
- un moyen 10 commandé de vidange de la capacité 8,
- une structure 11 de maintien d'une surface 7 orientée en vis à vis de la face frontale 5A de la paroi mince 5 pour recevoir au moins indirectement le substrat 2 en appui; lors de l'action de la paroi mince 5.

Lorsqu'un élément 3 de test est utilisé, il est de préférence placé contre celle 2A des faces opposées 2A, 2B du substrat 2 qui doit être appliquée vers la surface réceptrice 7 portée par la structure 11.

Le moyen 9 commandé d'injection d'un fluide sous pression dans la capacité 8 comprend :
- une source 9A de fluide sous pression,
- une conduite 9B qui relie la source 9A et la capacité 8,
- un organe 9C commandé de contrôle du passage du fluide sous pression dans la conduite 9B.

De manière préférentielle, la source de fluide sous pression est une source de fluide gazeux, tel de l'air et, cette source délivre le fluide sous une pression déterminée mais ajustable.

De préférence, le moyen 9 commandé comprend un organe 9D de visualisation de la pression du fluide sous pression.

De manière remarquable, le moyen 10 commandé de vidange de la capacité 8 est commandé par un moyen chronométrique 10A.

Par cela l'opérateur peut définir un temps d'application sous pression du substrat 2 et de l'élément absorbant 3.

Dans une forme de réalisation, la capacité 8 est constituée par le volume interne d'un caisson 8B substantiellement réalisé par une paroi rigide et localement par la paroi mince 5.

Par exemple, le caisson 8B est de forme sensiblement parallèlépipédique rectangle.

Selon une forme de réalisation, la paroi mince 5 consiste en une membrane 5 étanchement reliée au caisson 8B par sa périphérie.

Suivant une autre une forme de réalisation la face frontale 5A de la paroi mince 5 est constituée par l'une des faces d'une poche souple logée dans le caisson 8B (version non représentée).

Dans une forme de réalisation, la structure 11 de maintien d'une surface 7 orientée en vis à vis de la face frontale 5A de la paroi mince 5, comprend :
- une plaque 7A de matériau présentant une telle surface 7 orientée en vis à vis de la face frontale 5A de la paroi mince 5,
- une entretoise 11 qui porte la plaque 7A et à cet effet, s'étend au dessus de la membrane 5, pour porter ladite plaque à une distance correspondant sensiblement à l'épaisseur du substrat 2.

Avantageusement, l'entretoise 11 est constituée par un cadre qui s'étend à la périphérie et au dessus de la paroi mince 5.

De manière remarquable, la plaque 7A de matériau est articulée sur l'entretoise 11 entre deux positions dont,
- une position d'effacement qui permet l'accès à la face frontale de la paroi mince 5, notamment en vue de la mise en place ou du retrait du substrat 2, et
- une position d'application qui garantit le maintien de la surface réceptrice 7 de cette plaque 7A en vis à vis de la face frontale 5A de la paroi mince 5, de manière à recevoir au moins indirectement le substrat 2 en appui, lors de faction de la paroi mince 5.

Pour l'utilisation du dispositif de l'invention, de préférence, mais non limitativement, c'est celle dite réceptrice des faces du substrat qui, une fois équipée d'au moins un élément absorbant de test, est placée au contact de la plaque de matériau 7A.

De manière remarquable, la plaque de matériau 7A est réalisée en matériau transparent à au moins une certaine gamme d'ondes.

Par exemple, la plaque de matériau 7A est en matériau transparent à la lumière.

Cette particularité technique permet d'observer la face du substrat 2 et/ou de l'élément de test 3 qui se trouve appliqué contre la plaque 7A.

La plaque de matériau 7A peut avantageusement être courbe et avoir même une forme qui se rapproche de l'anatomie d'un individu.

Le caisson 8B ainsi que la paroi mince 5 seront bien évidemment conformés pour recevoir et s'appliquer sur la face courbe précitée.

Dans une variante de réalisation, le dispositif peut effectuer différents tests de mesure de temps d'absorption sous ou sans maintien de la pression hydrostatique.

A cet effet, la plaque 7A sera pourvu d'au moins une découpe surmontée d'une cheminée 100 dans laquelle sera introduit un liquide qui sera absorbé par le substrat absorbant.

Des électrodes placées à la base de la cheminée et à une distance suffisante de la substance absorbante, quelques millimètres au dessus du substrat, établissent un contact électrique tant qu'il y a du liquide dans cette cheminée.

A l'aide d'un matériel informatique, il sera possible de faire des multiples acquisitions en automatique par exemple pour reproduire les multiples mictions.

Le test dit d'acquisition et/ou de remouillage sera ainsi automatisé et performant grâce aux moyens mis en oeuvre et explicités plus haut.

## Revendications

1. Dispositif (1) pour la réalisation de tests sur des substrats absorbants (2), notamment tests de remouillage par application d'au moins l'une des faces opposées (2A, 2B) du dit substrat (2) contre une surface réceptrice (3A, 7),
ce dispositif étant **CARACTERISE en ce qu'**il comprend un moyen (4) dont la fonction est d'assurer l'application étroite du substrat (2) contre cette surface réceptrice (3A, 7), c'est à dire un moyen (4) à même de déformer élastiquement au moins ce substrat (2) pour qu'il s'appuie étroitement contre ladite surface réceptrice (3A, 7), le moyen (4) d'application étroite comprenant :
- une paroi mince (5) en matériau élastiquement déformable qui, d'étendue sensiblement supérieure à l'étendue de l'une quelconque des faces opposées (2A, 2B) du substrat (2) à tester, présente elle même deux faces opposées (5A, 5B) dont,
• une face (5A), dite frontale, destinée à coopérer au moins indirectement avec une face de substrat (2), et
• une face opposée (5B), dite dorsale, destinée à recevoir des actions d'application de la face frontale (5A) vers le substrat (2),
- un moyen (6) de production d'actions d'application contre la face dorsale de la paroi mince (5),
- une surface réceptrice (7) orientée pour recevoir au moins indirectement le substrat (2) en appui sous l'effet de la paroi mince (5),
- des moyens d'acquisition des données se rapportant aux caractéristiques du substrat absorbant (2) testé, notamment au remouillage.

2. Dispositif selon la revendication 1 **caractérisé en ce que** le moyen (4) d'application étroite est de type assurant une pression uniforme d'application du substrat absorbant (2) contre la surface réceptrice (3A, 7).

3. Dispositif selon la revendication 2 **caractérisé en ce qu'**il comprend :
- une capacité (8) définie par une paroi (8A) au moins localement constituée par la paroi mince (5),
- un moyen (9) commandé d'injection d'un fluide sous une pression déterminée dans cette capacité (8),
- un moyen (10) commandé de vidange de la capacité (8),
- une structure (11) de maintien d'une surface (7) orientée en vis à vis de la face frontale (5A) de la paroi mince (5) pour recevoir au moins indirectement le substrat (2) en appui, lors de l'action de la paroi mince (5).

4. Dispositif selon la revendication 3 **caractérisé en ce que** le moyen (9) commandé d'injection d'un fluide sous pression dans la capacité (8) comprend :
- une source (9A) de fluide sous pression,
- une conduite (9B) qui relie la source (9A) et la capacité (8),
- un organe (9C) commandé de contrôle du passage du fluide sous pression dans la conduite (9B).

5. Dispositif selon la revendication 4 **caractérisé en ce que** le moyen (10) commandé de vidange de la capacité (8) est commandé par un moyen chronométrique (10A).

6. Dispositif selon l'une quelconque des revendications 3 à 5 **caractérisé en ce que** la capacité (8) est constituée par le volume interne d'un caisson (8B) substantiellement réalisé par une paroi (8A), rigide et localement par la paroi mince (5).

7. Dispositif selon la revendication 6 **caractérisé en ce que** la paroi mince (5) consiste en une membrane (5) étanchement reliée au caisson (8B) par sa périphérie.

8. Dispositif selon l'une quelconque des revendications 3 à 7 **caractérisé en ce que** la structure (11) de maintien d'une surface (7) orientée en vis à vis de la face frontale (5A) de la paroi mince (5), comprend :
- une plaque (7A) de matériau présentant une telle surface (7) orientée en vis à vis de la face frontale (5A) de la paroi mince (5),
- une entretoise (11) qui porte la plaque (7A) et à cet effet, s'étend au dessus de la membrane (5), pour porter ladite plaque à une distance correspondant sensiblement à l'épaisseur du substrat (2) absorbant.

9. Dispositif selon la revendication 8 **caractérisé en ce que** la plaque (7A) de matériau est articulée sur l'entretoise (11) entre deux positions dont :
- une position d'effacement qui permet l'accès à la face frontale de la paroi mince (5), notamment en vue de la mise en place ou du retrait du substrat (2), et
- une position d'application qui garantit le maintien de la surface réceptrice (7) de cette plaque (7A) en vis à vis de la face frontale (5A) de la paroi mince (5), de manière à recevoir au moins indirectement le substrat (2) en appui, lors de l'action de la paroi mince (5).

## Patentansprüche

1. Vorrichtung zur Durchführung von Tests bei absorptionsfähigen Substraten (2), insbesondere von Benetzungstests, durch Auflegen mindestens einer der einander gegenüberliegenden Oberflächen (2A, 2B) des Substrats (2) auf eine Rezeptor-Oberfläche (3A, 7),
**dadurch gekennzeichnet, dass** die Vorrichtung umfasst
eine Einrichtung (4), deren Funktion darin besteht, das dichte Aufliegen des Substrats (2) auf der Rezeptoroberfläche (3A, 7) zu gewährleisten, das heißt eine Einrichtung (4), die ihrerseits mindestens das Substrat (2) elastisch verformen kann, sodass es dicht aufliegt auf der Rezeptoroberfläche (3A, 7), wobei die dicht aufliegende Einrichtung (4) umfasst:
- eine dünne Wand (5) aus einem elastisch verformbaren Material, deren Ausdehnung wesentlich größer ist als die Ausdehnung jeder der einander gegenüberliegenden Oberflächen (2A, 2B) des zu testenden Substrats (2), die ihrerseits zwei einander gegenüberliegende Oberflächen (5A, 5B) aufweist, von denen
• eine Oberfläche (5A), die so genannte frontale Oberfläche, dazu bestimmt ist, mindestens indirekt mit einer Oberfläche des Substrats (2) zu kooperieren, und
• eine entgegengesetzte Oberfläche (5B), die so genannte dorsale Oberfläche, dazu bestimmt ist, Auswirkungen der Auflage der frontalen Oberfläche (5A) auf dem Substrat (2) aufzunehmen,
- eine Einrichtung (6) zur Erzeugung von Wirkungen der Auflage auf der dorsalen Oberfläche der dünnen Wand (5),
- eine Rezeptoroberfläche (7), die so orientiert ist, dass sie mindestens indirekt das unter dem Einfluss der dünnen Wand (5) aufliegende Substrat (2) aufnehmen kann und
- Einrichtungen zur Erfassung von Daten, die sich auf die Eigenschaften des getesteten absorptionsfähigen Substrats (2), insbesondere auf die Benetzung, beziehen.

2. Vorrichtung nach Anspruch 1, **dadurch gekennzeichnet, dass** die dicht aufliegende Einrichtung (4) eine solche eines Typs ist, der die Erzeugung eines einheitlichen Auflagedruckes des absorptionsfähigen Substrats (2) auf der Rezeptoroberfläche (3A, 7) gewährleistet.

3. Vorrichtung nach Anspruch 2, **dadurch gekennzeichnet, dass** sie umfasst
- ein Aufnahmevolumen (8), das durch eine Wand (8A) begrenzt ist, die mindestens lokal aus der dünnen Wand (5) besteht,
- eine gesteuerte Einrichtung (9) zur Injektion eines Fluids bzw. einer Flüssigkeit unter einem vorgegebenen Druck in dieses Volumen (8),
- eine gesteuerte Einrichtung (10) zur Entleerung des Volumens (8) und
- eine Trägerstruktur (11) für eine Oberfläche (7), die so ausgerichtet ist, dass sie der frontalen Oberfläche (5A) der dünnen Wand (5) gegenüberliegt, für die mindestens indirekte Aufnahme des aufliegenden Substrats (2) unter der Einwirkung der dünnen Wand (5).

4. Vorrichtung nach Anspruch 3, **dadurch gekennzeichnet, dass** die gesteuerte Einrichtung (9) zur Injektion eines Fluids bzw. einer Flüssigkeit unter Druck in das Aufnahmevolumen (8) umfasst:
- eine Quelle (9A) für das unter Druck stehende Fluid (Flüssigkeit),
- eine Rohrleitung (9B), welche die Quelle (9A) und das Volumen (8) miteinander verbindet, und
- ein gesteuertes Organ (9C) zur Kontrolle des Stromes des unter Druck stehenden Fluids (Flüssigkeit) in der Rohrleitung (9B).

5. Vorrichtung nach Anspruch 4, **dadurch gekennzeichnet, dass** die gesteuerte Einrichtung (10) zur Entleerung des Volumens (8) mittels einer Zeitmesseinrichtung (10A) gesteuert ist.

6. Vorrichtung nach einem der Ansprüche 3 bis 5, **dadurch gekennzeichnet, dass** das Volumen (8) besteht aus dem Innenvolumen eines Behälters (8B), der im Wesentlichen besteht aus einer steifen Wand (8A) und lokal aus der dünnen Wand (5).

7. Vorrichtung nach Anspruch 6, **dadurch gekennzeichnet, dass** die dünne Wand (5) aus einer Membran (5) besteht, die an ihrem Umfang abdichtend mit dem Behälter (8B) verbunden ist.

8. Vorrichtung nach einem der Ansprüche 3 bis 7, **dadurch gekennzeichnet, dass** die Trägerstruktur (11) für eine Oberfläche (7), die so orientiert ist, dass sie der frontalen Oberfläche (5A) der dünnen Wand (5) gegenüberliegt, umfasst:
- eine Platte (7A) aus einem Werkstoff, die eine Oberfläche (7) aufweist, die so orientiert ist, dass sie der frontalen Oberfläche (5A) der dünnen Wand (5) gegenüberliegt,
- einen Rahmen (11), der die Platte (7A) trägt und zu diesem Zweck sich oberhalb der Membran (5) erstreckt, um die Platte in einem Abstand zu tragen, der im Wesentlichen der Dicke des absorptionsfähigen Substrats (2) entspricht.

9. Vorrichtung nach Anspruch 8, **dadurch gekennzeichnet, dass** die Werkstoffplatte (7A) auf dem Rahmen (11) zwischen zwei Positionen frei beweglich ist:
- einer Wegnahme-Position, die Zugang zu der frontalen Oberfläche der dünnen Wand (5) verschafft, insbesondere um das Substrat (2) einzulegen oder wegzunehmen, und
- eine Auflage-Position, die das Andrücken der Rezeptoroberfläche (7) dieser Platte (7A) an die frontale Oberfläche (5A) der dünnen Wand (5) gewährleistet, um mindestens indirekt das aufliegende Substrat (2) aufzunehmen unter der Einwirkung der dünnen Wand (5).

## Claims

1. Device (1) for carrying out tests on absorbent substrates (2), particularly remoistening tests, by applying at least one of the opposite faces (2A, 2B) of the said substrate (2) against a receiving surface (3A, 7), this device being **characterised in that** it comprises a means (4) which has the purpose of ensuring close application of the substrate (2) against this receiving surface (3A, 7), i.e. a means (4) capable of elastically deforming at least this substrate (2) so that it is closely supported against the said receiving surface (3A, 7), the means of close application comprising:
- a thin wall (5) in an elastically deformable material which, of a more or less greater extent than the extent of any one of the opposite faces (2A, 2B) of the substrate (2) to be tested, has itself two opposite faces (5A, 5B) in which
- one face (5A), called the front face, is intended to work in conjunction at least indirectly with one face of the substrate (2), and
- an opposite face (5B), called the back face, is intended to receive actions for applying the front face (5A) towards the substrate (2);
- a means (6) of producing actions of application against the back face of the thin wall (5);
- a receiving surface (7) directed to take at least indirectly the substrate (2) supported under the effect of the thin wall (5);
- means of collecting data relating to the features of the absorbent substrate (2) tested, particularly when remoistening.

2. Device according to Claim 1, **characterised in that** the means (4) of close application are of a type which ensures uniform pressure of application of the absorbent substrate (2) against the receiving surface (3A, 7).

3. Device according to Claim 2, **characterised in that** it comprises:
- a container (8) defined by a wall (8A) at least locally consisting of the thin wall (5);
- a controlled means (9) of injecting a fluid under a particular pressure into this container (8);
- a controlled means (10) of draining the container (8);
- a structure (11) for keeping a surface (7) directed facing the front face (5A) of the thin wall (5) to take at least indirectly the supported substrate (2) when the thin wall (5) acts.

4. Device according to Claim 3, **characterised in that** the controlled means (9) of injecting a pressurised fluid into the container (8) comprises:
- a source (9A) of pressurised fluid;
- a line (9B) which connects the source (9A) and the container (8);
- a controlled unit (9C) to control the flow of the pressurised fluid in the line (9B).

5. Device according to Claim 4, **characterised in that** the controlled means (10) of draining the container (8) is operated by chronometric means (10A).

6. Device according to any one of the Claims 3 to 5, **characterised in that** the container (8) consists of the internal space of a casing (8B) substantially made from a rigid wall (8A) and locally by the thin wall (5).

7. Device according to Claim 6, **characterised in that** the thin wall (5) consists of a diaphragm (5) sealed to the casing (8B) along its periphery.

8. Device according to any one of the Claims 3 to 7, **characterised in that** the structure (11) holding a surface (7) directed facing the front face (5A) of the thin wall (5), comprises:
- a plate (7A) which has such a surface (7) directed facing the front face (5A) of the thin wall (5);
- a spacer (11) which carries the plate (7A) and, for this purpose, extends above the diaphragm (5), to carry the said plate at a distance corresponding more or less to the thickness of the absorbent substrate (2).

9. Device according to Claim 8, **characterised in that** the material plate (7A) is hinged to the spacer (11) between two positions so that there is:
- a withdrawal position which allows access to the front face of the thin wall (5), particularly with a view to putting the substrate (2) in place or withdrawing it, and
- an application position which ensures that the receiving surface (7) of this plate (7A) is kept facing the front face (5A) of the thin wall (5) in such a way as to receive, at least indirectly, the supported substrate (2) when the thin wall (5) acts.
